# FASCICULE DE BREVET EUROPEEN

(11) **EP 0 553 585 B1**
(45) Date de publication et mention de la délivrance du brevet: **29.11.1995**
(21) Numéro de dépôt: 92420487.8
(22) Date de dépôt: 30.12.1992
(51) Int. Cl.: A61F 2/38

(54) **Prothèse totale du genou intracondylienne**
Intrakondyläre Knie-Totalprothese
Intracondylar total knee prosthesis

(30) Priorité: 31.12.1991 FR 9116498
(43) Date de publication de la demande: 04.08.1993
(73) Titulaire: La Société F.I.I., 43240 Saint-Just-Malmont (FR)
(72) Inventeur: Reigner, Bernard, 72000 Le Mans (FR)
(74) Mandataire: Dupuis, François

(56) Documents cités:
- EP-A- 0 174 531
- FR-A- 2 330 375
- FR-A- 2 601 873

## Description

D'une manière parfaitement connue, ce type de prothèse comprend un élément fémoral et un élément tibial accouplés de manière à assurer une flexion de grande amplitude et une rotation limitée du fémur par rapport au tibia. Plus particulièrement, l'invention concerne une prothèse du type de celle décrite dans la demande de brevet FR 2601873, dont le demandeur de la présente est également titulaire.

Pour l'essentiel, dans ce type de prothèse, l'élément fémoral est en appui par des patins condyliens sur un plateau tibial, généralement en polyéthylène. L'élément fémoral reçoit un axe transversal d'articulation monté à libre rotation par rapport à une tige cylindrique, engagée dans un fourreau de l'élément tibial, pour permettre, en combinaison, des mouvements de flexion et de rotation. La rotation de l'élément fémoral par rapport à l'élément tibial est limitée, notamment par des agencements que présente une partie de l'élément tibial.

Selon l'invention, on a voulu améliorer les caractéristiques de cette prothèse. Notamment il est apparu nécessaire de supprimer, ou tout au moins de limiter, le mouvement de piston existant entre les éléments fémoral et tibial, résultant de l'engagement de la tige cylindrique dans le fourreau de l'élément fémoral. Ces dispositions s'avèrent importantes pour supprimer tout risque de luxation des deux éléments de prothèse.
Or, cette faculté de supprimer le mouvement de piston entre l'élément fémoral et l'élément tibial, est possible, étant donné que la limitation de rotation entre ces deux éléments n'est pas faite par la mise en tension des ligaments latéraux, qui nécessite un tel mouvement de piston.

Le problème que se propose de résoudre l'invention est de limiter le mouvement de piston tout en tenant compte de la limitation en rotation de l'élément fémoral par rapport à l'élément tibial.
Pour résoudre un tel problème, les moyens sont constitués par une vis engagée dans l'épaisseur du plateau tibial et coopérant avec une gorge formée sur une partie seulement de la circonférence de la tige, selon un arc correspondant très sensiblement à la limitation de rotation de l'élément fémoral par rapport à l'élément tibial.
La vis coopère avec la gorge, par l'intermédiaire d'un pion rapporté disposé coaxialement à ladite vis.

Compte-tenu de la limitation de l'élément fémoral par rapport à l'élément tibial, la gorge est formée sur une partie seulement de la circonférence de la tige, selon un arc correspondant très sensiblement à la limitation de rotation de l'élément fémoral par rapport à l'élément tibial.
L'arc de cercle délimite un angle de très sensiblement 30°.

Pour résoudre le problème posé de permettre un mouvement de piston limité entre l'élément fémoral et l'élément tibial, l'épaisseur de la gorge est supérieur au diamètre du plot.

Avantageusement, la vis est en polyéthylène, le plot étant métallique.

L'invention est exposée, ci-après plus en détail à l'aide des dessins annexés, dans lesquels :

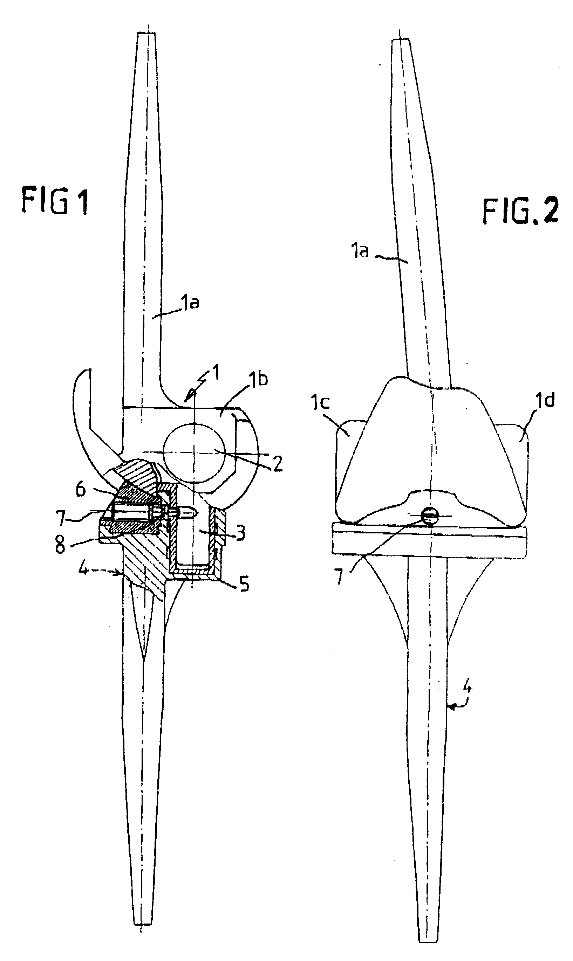

- La figure 1 est une vue en coupe longitudinale de la prothèse selon l'invention.
- La figure 2 est une vue de face correspondant à la figure 1.
- La figure 3 est à une échelle plus importante, une vue en coupe montrant le détail d'accouplement entre l'élément fémoral et l'élément tibial.
- La figure 4 est à grande échelle, une vue partielle en coupe de la tige dans son fourreau.

D'une manière connue, l'élément fémoral (1) comprend une tige d'ancrage (1a) destinée à être engagée dans le canal médullaire du fémur. L'élément fémoral (1) présente une saillie centrale creuse (1b) dont les parois transversales sont agencées pour le montage à libre rotation, d'un axe (2). Sur l'axe d'articulation (2), tourillonne une tige cylindrique (3) destinée à coopérer avec l'élément tibial (4). Notamment, la tige (3) est engagée dans un fourreau (5) que présente l'élément tibial (4).

De part et d'autre de l'échancrure (1b) l'élément fémoral (1) présente des patins condyliens (1c) (1d) coopérant en appui avec un plateau tibial en polyéthylène (6), que présente l'élément (4). Le montage de l'élément fémoral (1) par rapport à l'élément tibial (4), compte-tenu des dispositions de l'axe (2) et de la tige (3) permet les mouvements de flexion et de rotation des deux éléments (1) et (4).
Ces dispositions ne sont pas décrites en détail car elles sont parfaitement connues pour un homme du métier et ne font pas partie de l'objet spécifique de l'invention. Par ailleurs, de telles dispositions sont clairement décrites et illustrées dans le brevet précité FR 2601873.

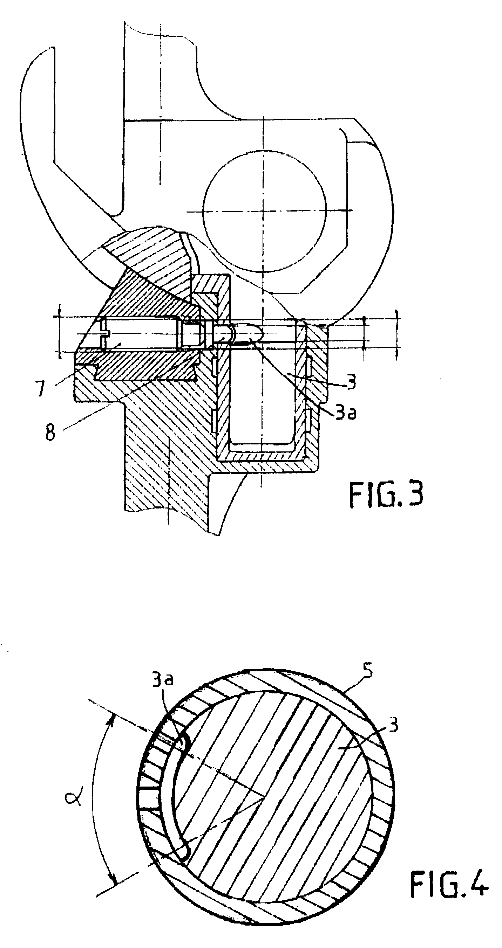

Selon l'invention, le plateau tibial (6) et la tige (3) présentent des moyens complémentaires pour limiter le mouvement de déplacement vertical de l'élément fémoral par rapport à l'élément tibial, tout en permettant leur rotation partielle. Dans ce but, une vis (7), est engagée dans l'épaisseur du plateau tibial (6) selon son axe médian et à partir de sa face antérieure. Cette vis (7) coopère par l'intermédiaire d'un plon rapporté (8) avec une gorge (3a) que présente la tige (3) (figure 3).

Comme le montre la figure 4, la gorge (3a) est formée sur une partie seulement de la circonférence de la tige (3), selon un arc de cercle (α) de très sensiblement 30° correspondant à la limitation de rotation entre l'élément fémoral et l'élément tibial.

On rappelle que cette limitation à 30° correspond à une limitation de la rotation tibiale à 15°, de part et d'autre de la position neutre. Ce contrôle de rotation peut s'effectuer par un usinage particulier du plateau tibial, notamment au niveau du fourreau (5). L'épaisseur de la gorge (3a) est supérieur au diamètre du plot (8) , permettant ainsi un mouvement de déplacement vertical très limité de l'ordre de quelques dixièmes de millimètres de l'élément fémoral (1) par rapport à l'élément tibial (4).

Bien évidemment, la vis (7) et le plot (8) sont mis en place après impaction de l'élément fémoral et de l'élément tibial et après accouplement desdits éléments, notamment après engagement de la tige (3) dans le fourreau (5). La cinématique de cette prothèse demeure réalisée d'une façon connue, c'est-à-dire par la combinaison d'un mouvement de flexion et de rotation limité et guidé.

Les avantages ressortent bien de la description, en particulier on souligne et on rappelle :
- La suppression du mouvement de piston entre l'élément fémoral et l'élément tibial, évitant ainsi tout risque de luxation.
- La simplicité de réalisation.

Enfin, on souligne que la vis (7) est réalisée en polyéthylène, tandis que le pion de centrage (8) est en métal.

## Revendications

1. Prothèse totale du genou du type intracondylienne, comprenant un élément fémoral (1) en appui par des patins condyliens (1c) (1d) sur un plateau tibial (6) que présente un élément tibial (4), l'élément fémoral (1) recevant un axe transversal d'articulation (2) monté à libre rotation par rapport à une tige cylindrique (3), engagée dans un fourreau (5) de l'élément tibial (4), pour permettre, en combinaison, des mouvements de flexion et de rotation, la rotation de l'élément fémoral (1) par rapport à l'élément tibial (4) étant limitée, le plateau tibial (6) et la tige cylindrique (3) présentant des moyens complémentaires (7) (8) (3a) aptes à limiter le mouvement de déplacement vertical relatif de l'élément fémoral par rapport à l'élément tibial, caractérisée en ce que les moyens sont constitués par une vis (7) engagée dans l'épaisseur du plateau tibial (6) et coopérant avec une gorge (3a) formée sur une partie seulement de la circonférence de la tige (3), selon un arc (α) correspondant très sensiblement à la limitation de rotation de l'élément fémoral (1) par rapport à l'élément tibial (4).

2. Prothèse selon la revendication 1, caractérisée en ce que la vis (7) coopère avec la gorge (3a), par l'intermédiaire d'un pion rapporté (8) disposé coaxialement à ladite vis (7).

3. Prothèse selon la revendication 1, caractérisée en ce que l'arc de cercle délimite un angle (α) de très sensiblement 30°.

4. Prothèse selon la revendication 2, caractérisée en ce que l'épaisseur de la gorge (3a) est supérieur au diamètre du pion (8).

5. Prothèse selon la revendication 2, caractérisée en ce que la vis (7) est en polyéthylène, le pion (8) étant métallique.

## Claims

1. A total knee prosthesis of intracondylar type, consisting of a femoral element (1) bearing by means of condylar runners (1c) (1d) on a tibial plateau (6) prolonged by a tibial element (4), said femoral element (1) being equipped with a transverse articulation axle (2) rotating freely with respect to a cylindrical rod (3) held in a casing (5) in the tibial element (4), allowing combined flexion and rotation movements, rotation of the femoral element (1) with respect to the tibial element (4) being limited, said tibial plateau (6) and said cylindrical rod (3) having additional means (7) (8) (3a) capable of limiting relative vertical displacement of said femoral element with respect to said tibial element, characterized in that said means consist of one screw (7) inserted into the thickness of said tibial plateau (6) and fitting into a groove (3a) running over a portion of the circumference of said rod (3) and forming an arc (α) limiting the rotation of said femoral element (1) with respect to said tibial element (4).

2. A prosthesis according to claim 1, characterized in that said screw (7) fits into said groove (3a) by means of a tip (8) fixed coaxially to said screw (7).

3. A prosthesis according to claim 1, characterized in that the arc of the circle defines an angle (α) of very nearly 30°.

4. A prosthesis according to claim 2, characterized in that the width of said groove (3a) is greater than the diameter of said tip (8).

5. A prosthesis according to claim 2, characterized in that said screw (7) is made of polyethylene and said tip (8) is made of metal.

## Patentansprüche

1. Intrakondyläre Totalendoprothese des Knies bestehend aus einem femoralen Element (1), das über Kondylenschuhe (1c) (1d) auf einem Tibiaplateau (6) eines tibialen Elements (4) aufliegt, wobei das femorale Element (1) eine querverlaufende Gelenkachse (2) aufnimmt, die frei drehbar im Verhältnis zu einer zylinderförmigen Stange (3) angebracht ist, welche in einer Hülle (5) des tibialen Elements (4) sitzt, um kombinierte Beuge- und Drehbewegungen zu ermöglichen, wobei die Drehung des femoralen Elements (1) gegenüber dem tibialen Element (4) begrenzt ist, da das Tibiaplateau (6) und die Zylinderstange (3) sich ergänzende Vorkehrungen (7) (8) (3a) aufweisen, die geeignet sind, die relative senkrechte Bewegung des femoralen Elements gegenüber dem tibialen Element zu begrenzen, dadurch gekennzeichnet, daß diese Vorkehrungen aus einer Schraube (7) bestehen, die in das Tibiaplateau (6) eingeführt ist und mit einer Nut (3a) zusammenwirkt, die in nur einem Teil des Umfangs der Stange (3) verläuft, und zwar in Form eines Bogens (α), der ziemlich genau der Begrenzung der Drehbewegung des femoralen Elements (1) gegenüber dem tibialen Element (4) entspricht.

2. Prothese nach Anspruch 1, dadurch gekennzeichnet, daß die Schraube (7) über ein koaxial zur Schraube (7) angesetztes Zentrierstück (8) mit der Nut (3a) zusammenwirkt.

3. Prothese nach Anspruch 1, dadurch gekennzeichnet, daß der Kreisbogen einen Winkel (α) von ziemlich genau 30° begrenzt.

4. Prothese nach Anspruch 2, dadurch gekennzeichnet, daß die Nut (3a) größer ist als der Durchmesser des Zentrierstücks (8).

5. Prothese nach Anspruch 2, dadurch gekennzeichnet, daß die Schraube (7) aus Polyethylen und das Zentrierstück (8) aus Metall besteht.
